# EUROPEAN PATENT APPLICATION

(11) **EP 4 248 738 A1**
(43) Date of publication of application: **27.09.2023**
(21) Application number: 21894712.5
(22) Date of filing: 18.11.2021
(51) Int. Cl.: A01H 6/82, A01H 1/00, A01H 5/00, A01H 5/10, C12N 15/29, C12Q 1/6895

(54) **CYTOPLASMIC MALE STERILE PLANT OF GENUS PETUNIA, INTERGENERIC HYBRID PLANT THEREOF, AND METHOD OF PRODUCING SAME**

(30) Priority: 20.11.2020 JP 2020193820
(71) Applicant: Sakata Seed Corporation, Yokohama-shi Kanagawa 224-0041 (JP)
(72) Inventor: HORIUCHI, Shingo, Yokohama-shi, Kanagawa 224-0041 (JP); SUZUKI, Takao, Yokohama-shi, Kanagawa 224-0041 (JP); TORII, Akihiro, Yokohama-shi, Kanagawa 224-0041 (JP); IZUMIDA, Atsushi, Yokohama-shi, Kanagawa 224-0041 (JP)
(74) Representative: Bandpay & Greuter
(86) International application number: PCT/JP2021/042382
(87) International publication number: WO 2022/107839

(57) **Abstract**

Disclosed is a cytoplasmic male sterile plant of the genus Petunia having, in the mitochondrial genome thereof, a DNA molecule originated from the mitochondrial genome of a tobacco plant, or a hybrid plant with the cytoplasmic male sterile plant of the genus Petunia, or a progeny of the cytoplasmic male sterile plant of the genus Petunia or the hybrid plant. The cytoplasmic male sterile plant of the genus Petunia is a stable line in which the growth ability of young seedlings is improved and which is not susceptible to fertility restoration, and further, achieves diversification of available CMS cytoplasms.

## Description

### [Technical Field]

The present invention relates to a cytoplasmic male sterile plant of the genus Petunia, or a hybrid plant with the cytoplasmic male sterile plant of the genus Petunia, or a progeny thereof. The present invention also relates to a method for producing the same.

### [Background Art]

Petunia is a general term for horticultural species of the genus Petunia and the family Solanaceae, and about 16 species of the genus Petunia are native to the American continent. Petunia of a horticultural variety are referred to as Petunia x hybrida and originated from an interspecific hybrid of Petunia axillaris and Petunia integrifolia. Since breeding of petunia started in the United Kingdom in the 1830s, varieties having various flower colors, flower diameters, and plant habit have been developed, and at present, petunia is one of the most important flowers and ornamental items for flower beds and potting worldwide. (NPTL 1).

In general, plant varieties include phenotypically uniform open pollinated varieties and first filial generation hybrid varieties (hereinafter referred to as "F1"), and F1 varieties are prevalent among major crops. F1 varieties have great advantages, due to heterosis, such as vigorous growth, fast growth, and increased yield. Furthermore, F1 varieties can be expected to be improved in environmental adaptability such as cold resistance and heat resistance due to their vigorous growth.

Moreover, because individuals of F1 varieties have nearly the same genotype, their phenotype exhibits an extremely high degree of uniformity despite having heterozygousity. Therefore, the marketability of these varieties is increased. Furthermore, because useful traits controlled by dominant genes, can be accumulated in the parent lines of F1 varieties, rapid breeding is possible.

Due to these advantages, F1 varieties represent the majority of cultivars in major crops.

In the production of seeds of F1 varieties, self-fertilized (inbred) lines are generally used as parents, and seed parents and pollen parents are selected from combinations that have a large effect of heterosis.

For seed parents, emasculation is needed to prevent self-fertilization, but manual emasculation requires a great deal of labor. Hence, a line having cytoplasmic male sterility (hereinafter referred to as "CMS"), which is genetically male sterile, is useful as the seed parent to eliminate manual emasculation so F1 seeds can be produced economically in a large quantity. Commercial production systems for F1 seeds using CMS have been established for sunflower, sugar beet, wheat, carrot, onion, leek, cabbage, broccoli, cauliflower, radish, Chinese cabbage, and other crops.

A cytoplasmic male sterile line of Petunia was obtained by crossing petunia (P. hybrida) as a pollen parent and an unknown wild species (presumed to be P. axillaris, P. integrifolia or P. parodii) as a seed parent by H.L. Everett and W.H. Gabelman. This information suggests that the cytoplasmic male sterile line of Petunia is a heterologous cytoplasmic line (alloplasmic line) and that a combination of nucleus and cytoplasm resulted in cytoplasmic male sterility.

However, records disclosing the origin of the CMS in Petunia are not clear, and the origin of the CMS remains unknown. It also remains possible that the mitochondrial DNA (mtDNA) encoding CMS was generated through rearrangements induced by combining a nuclear genome of one species with a mitochondrial genome of another species, not through true heterologous cytoplasmic male sterility. While the CMS of Petunia was introduced into Petunia by backcrossing and spread into other breeding families, only one CMS has so far been used. The causative gene of Petunia CMS was sequenced by Young and Hanson in 1987 and is called the pcf gene (Petunia CMS-associated fused gene) (NPTL 2).

While there are many academic CMS research cases (NPTL 3), in practical use CMS lines caused by the pcf gene of Petunia (hereinafter referred to as "pcf-CMS") have been reported to be associated with various deleterious traits such as arrested development of flower buds, reduction in flower size, and delay of flowering. These deleterious traits vary depending on the genotype of the line to be converted to CMS, and it is possible to use a line in which the expression of deleterious traits is minor. However, this has the disadvantage that the number of parent lines that can be used is limited, and careful trialing is required before the product is released.

Male sterility in the "pcf-CMS" line is known to be restored to fertility by a single dominant gene or a plurality of fertility restoration genes, and in some lines it may be difficult to introduce male sterility. Furthermore, it is known that the male sterility of the "pcf-CMS" line is restored to fertility by environmental changes, even when the male sterility seems to be stable (NPTL 3).

As described above, the "pcf-CMS" line of Petunia has many difficult problems such that the varieties in which the "pcf-CMS" line is used is limited to Gioconda, Capri series and the like of Farao Seeds, and development of new CMS lines having different origins of CMS is desired.

### [Citation List]

### [Non Patent Literature]

NPTL 1: Agricultural Technology System, Flowers and Ornamental Plants Edition, Vol. 8, 1 · 2-annual plants Petunias, pp. 372-4 to 372-9, issued by Rural Culture Association Japan
NPTL 2: J.D. Gillman et al, "Cytoplasmic Male Sterility and Fertility Restoration in Petunia", chapter 6, pp.107-129, Petunia, DOI (2009)
NPTL 3: M.L.K. Kaul, Male Sterility in Higher Plants, pp.809-810, Springer-Verlag, (1998)

### [Summary of Invention]

### [Technical Problem]

We have studied breeding utilization of a known "pcf-CMS" line of petunia and have found that deterioration in growth ability in young seedlings is one of the major problems, in addition to various points known in the related art such as arrested development of flower buds and delay of flowering.

It is known that the male sterility of the conventional pcf-CMS line is restored to fertility by a single dominant gene or a plurality of fertility restoration genes.

Further, in the known pcf-CMS line, an unknown wild species of the genus Petunia is used, and the known CMS line of Petunia is the only CMS line which is used in practice. Dependence on a single cytoplasm is of concern for genetic vulnerability, as is known in the case where maize F1 varieties using T-type CMS were severely damaged by T-race of southern leaf blight. Therefore, diversification of CMS cytoplasm has been desired.

In view of the problem that the growth ability in young seedlings of the known "pcf-CMS" line is deteriorated as described above, another object of the present invention is to provide a novel cytoplasmic male sterile line in which the growth ability in young seedlings is not deteriorated, and a stable cytoplasmic male sterile line that is not susceptible to fertility restoration. It is also an object to thereby achieve diversification of CMS cytoplasm. Further, an object of the present invention is to provide a method for producing F1 seeds of Petunia using the novel cytoplasmic male sterile line.

### [Solution to Problem]

We have succeeded in constructing a novel cytoplasmic male sterile Petunia in which the growth ability in young seedlings is not deteriorated, i.e., the growth ability of a young seedling is improved, by carrying out asymmetric protoplast fusion using Nicotiana suaveolens as a cytoplasm donor parent and petunia having a normal cytoplasm as a cytoplasm acceptor parent. Further, it has been found that F1 seeds of petunia in which the growth ability in young seedlings is not deteriorated are obtained by using the novel cytoplasmic male sterile Petunia. In addition, the obtained cytoplasmic male sterile Petunia used a cytoplasm originated from a tobacco plant, and since there is also no fertility restoration gene for the cytoplasm from the tobacco plant in plants of the genus Petunia, and no fertility restoration genes of the plants of the genus Petunia are known to restore fertility with the tobacco cytoplasm, it is considered a fact that the cytoplasmic male sterility is stabilize. Therefore, we have succeeded in obtaining a stable cytoplasmic male sterile line that is not susceptible to fertility restoration. Furthermore, diversification of CMS cytoplasm is also achieved, as the line differs from known cytoplasmic male sterile lines.

The present invention has been made on the basis of these findings.

According to the present invention, the following inventions are provided.
<1> A cytoplasmic male sterile plant of the genus Petunia comprising, in a mitochondrial genome thereof, a DNA molecule originated from a mitochondrial genome of a tobacco plant, or a hybrid plant with the cytoplasmic male sterile plant of the genus Petunia, or a progeny thereof.
<2> The cytoplasmic male sterile plant of the genus Petunia or the hybrid plant with the cytoplasmic male sterile plant of the genus Petunia, or the progeny thereof according to <1>, wherein the tobacco plant is Nicotiana suaveolens.
<3> The cytoplasmic male sterile plant of the genus Petunia or the hybrid plant with the cytoplasmic male sterile plant of the genus Petunia, or the progeny thereof according to <1> or <2>, wherein the cytoplasmic male sterile plant of the genus Petunia is originated from Petunia hybrida or an interspecific hybrid plant thereof.
<4> The cytoplasmic male sterile plant of the genus Petunia or the hybrid plant with the cytoplasmic male sterile plant of the genus Petunia, or the progeny thereof according to any one of <1> to <3>, wherein the cytoplasmic male sterile plant of the genus Petunia is originated from one produced by asymmetric protoplast fusion using a tobacco plant as a cytoplasm donor parent.
<5> The cytoplasmic male sterile plant of the genus Petunia or the hybrid plant with the cytoplasmic male sterile plant of the genus Petunia, or the progeny thereof according to any one of <1> to <4>, wherein the hybrid plant with the cytoplasmic male sterile plant of the genus Petunia is originated from an intergeneric hybrid plant between a plant of the genus Petunia and a plant of the genus Calibrachoa.
<6> The cytoplasmic male sterile Petunia plant or the hybrid plant with the cytoplasmic male sterile Petunia plant, or the progeny thereof according to any one of <1> to <5>, comprising a mitochondrial genome originated from a plant identified by Accession No. FERM BP-22398.
<7> The cytoplasmic male sterile Petunia plant or the hybrid plant with the cytoplasmic male sterile Petunia plant, or the progeny thereof according to any one of <1> to <6>, wherein at least one of mitochondrial DNA regions is a Nicotiana suaveolens type where the regions are identified by a mitochondrial genome marker using one or more primers selected from the group consisting of primers contained in a primer set having nucleotide sequences set forth in SEQ ID NOs: 59 and 60 and a primer set having nucleotide sequences set forth in SEQ ID NOs: 67 and 68.
<8> The cytoplasmic male sterile Petunia plant or the hybrid plant with the cytoplasmic male sterile Petunia plant, or the progeny thereof according to any one of <1> to <7>, comprising a mitochondrial genome of a plant identified by Accession No. FERM BP-22398.
<9> The cytoplasmic male sterile Petunia plant or the hybrid plant with the cytoplasmic male sterile Petunia plant, or the progeny thereof according to any one of <1> to <7>, which is identified by Accession No. FERM BP-22398.
<10> The cytoplasmic male sterile Petunia plant or the hybrid plant with the cytoplasmic male sterile Petunia plant, or the progeny thereof according to any one of <1> to <9>, which is produced by asymmetric protoplast fusion using, as a cytoplasm donor parent, a cytoplasmic male sterile Petunia plant or a hybrid plant with a cytoplasmic male sterile Petunia plant, having a mitochondrial genome of a plant identified by Accession No. FERM BP-22398, and using, as a cytoplasm acceptor parent, a Petunia plant or a hybrid plant with a Petunia plant, having a normal cytoplasm.
<11> A part of a plant body of the cytoplasmic male sterile Petunia plant or the hybrid plant with the cytoplasmic male sterile Petunia plant, or the progeny thereof as defined in any one of <1> to <10>.
<12> A seed of the cytoplasmic male sterile Petunia plant or the hybrid plant with the cytoplasmic male sterile Petunia plant, or the progeny thereof as defined in any one of <1> to <10>.
<13> A mitochondrial genome contained in the cytoplasmic male sterile plant of the genus Petunia or the hybrid plant with the cytoplasmic male sterile plant of the genus Petunia, or the progeny thereof as defined in any one of <1> to <10>, the part of the plant body as defined in <11>, or the seed as defined in <12>.
<14> A method for producing a cytoplasmic male sterile plant of the genus Petunia or a hybrid plant with the cytoplasmic male sterile plant of the genus Petunia, or a progeny thereof, the method comprising a step of carrying out asymmetric protoplast fusion using a tobacco plant as a cytoplasm donor parent and using a plant of the genus Petunia or a hybrid plant with a plant of the genus Petunia, having a normal cytoplasm, as a cytoplasm acceptor parent.
<15> The production method according to <14>, wherein the tobacco plant is Nicotiana suaveolens.
<16> A method for producing a cytoplasmic male sterile plant of the genus Petunia having an improved mitochondrial genome or a hybrid plant with the cytoplasmic male sterile plant of the genus Petunia, or a progeny thereof, the method comprising a step of carrying out asymmetric protoplast fusion using a cytoplasmic male sterile plant of the genus Petunia having, in a mitochondrial genome thereof, a DNA molecule originated from a mitochondrial genome of a tobacco plant, or a hybrid plant with the cytoplasmic male sterile plant of the genus Petunia, or a progeny thereof as a cytoplasm donor parent and using a plant of the genus Petunia or a hybrid plant with a plant of the genus Petunia, having a normal cytoplasm, as a cytoplasm acceptor parent.
<17> The production method according to any one of <14> to <16>, wherein the cytoplasmic male sterile plant of the genus Petunia is originated from Petunia hybrida or an interspecific hybrid plant thereof.
<18> The production method according to any one of <14> to <17>, wherein the hybrid plant with the cytoplasmic male sterile plant of the genus Petunia is originated from an intergeneric hybrid plant between a plant of the genus Petunia and a Calibrachoa plant.
<19> A method for producing a first filial generation seed, comprising the step of: carrying out crossbreeding using, as a seed parent, the cytoplasmic male sterile plant of the genus Petunia or the hybrid plant with the cytoplasmic male sterile plant of the genus Petunia, or the progeny thereof as defined in any one of <1> to <10> and using, as a pollen parent, a plant of the genus Petunia capable of being crossbred with the above plant or an intergeneric hybrid originated from the plant of the genus Petunia and then producing a first filial generation seed from the seed parent after the crossbreeding.
<20> A first filial generation seed produced by a method as defined in <19>, a first filial generation plant grown from the seed or a progeny thereof, or a part of a plant body of the first filial generation plant or the progeny thereof.
<21> A method for producing a plant of the genus Petunia exhibiting cytoplasmic male sterility and an intergeneric hybrid plant originated therefrom, the method comprising the steps of: carrying out recurrent backcrossing of an arbitrary plant of the genus Petunia and an intergeneric hybrid plant originated therefrom to a cytoplasmic male sterile plant of the genus Petunia or a hybrid plant with the cytoplasmic male sterile plant of the genus Petunia, or a progeny thereof as defined in any one of <1> to <10> to achieve cytoplasmic replacement.
<22> A primer set comprising a primer having a nucleotide sequence set forth in SEQ ID NO: 59 and a primer having a nucleotide sequence set forth in SEQ ID NO: 60.
<23> A primer set comprising a primer having a nucleotide sequence set forth in SEQ ID NO: 67 and a primer having a nucleotide sequence set forth in SEQ ID NO: 68.

### [Results of Invention]

According to the present invention, it is possible to provide a cytoplasmic male sterile plant of the genus Petunia in which deterioration in the growth ability of young seedlings, which is found in known ones, is not observed, i.e., the growth ability of a young seedling is improved. With the novel cytoplasmic male sterile plant of the genus Petunia according to the present invention, it is possible to efficiently produce F1 seeds of a plant of the genus Petunia in which the growth ability of a young seedling is improved.

It is known that male sterility of a known pcf-CMS line is restored to fertility by a single dominant gene or a plurality of fertility restoration genes, and since the cytoplasmic male sterile line according to the present invention uses a cytoplasm originated from a tobacco plant, and it is considered that the known fertility restoration genes of a Petunia plant do not function, and there is no fertility restoration gene of the cytoplasm originated from the tobacco plant in the plant of the genus Petunia. Therefore, according to the present invention, it is possible to provide a stable cytoplasmic male sterile line that does not cause fertility restoration.

In addition, in the known pcf-CMS line, an unknown wild species of the genus Petunia is used, and the known CMS line of Petunia is the only CMS line which is used in practice. Dependence on a single cytoplasm is of concern for genetic vulnerability, as is known in the case where maize F1 varieties using T-type CMS were severely damaged by T-race of southern leaf blight. Therefore, diversification of CMS cytoplasm has been desired. According to the present invention, a new petunia cytoplasmic male sterile line different from the known petunia cytoplasmic male sterile line is provided, so that "diversification of CMS cytoplasm" can be realized. As a result, it is possible to develop more diverse new varieties than before.

In addition, according to the asymmetric back protoplast fusion method of the present invention, it is possible to improve a cytoplasm, particularly a mitochondrial genome, of a plant of the genus Petunia and an intergeneric hybrid plant thereof.

### [Brief Description of Drawings]

Fig. 1 shows flower morphology of a novel petunia cytoplasmic male sterile line "P4".
Fig. 2 shows the results of comparing the growth ability of a young seedling between a recurrently backcrossed progeny (BC3) of the novel petunia cytoplasmic male sterile line "P4" (left group in the figure) and its recurrent parent line (right group in the figure). In the figure, the growth ability of young seedlings in the recurrently backcrossed progeny of "P4" is deteriorated as compared with the parent line.
Fig. 3 is a photograph which shows comparison of a multiflora petunia parent line "Pt3" having a red flower color with a novel petunia cytoplasmic male sterile line "Q15" selected by recurrent backcrossing using "Pt3" as a pollen parent (recurrent parent).
Fig. 4 shows photographs of anther morphology of each of "Pt3" having a normal cytoplasm, a cytoplasmic male sterile line "pcf-CMS" produced by recurrent backcrossing (BC7) of "Pt3", and "Q15" taken with a stereomicroscope.
Fig. 5 is a photograph showing the difference in the growth ability when seedlings were raised in an artificial climate chamber set to a day temperature of 22°C, a night temperature of 15°C and a lighting time of 16 hours using "Pt3", a novel CMS line "Q15" produced by seven recurrent backcrossings (BC7) using "Pt3" as a recurrent parent and "pcf-CMS", which is the known CMS line.
Fig. 6 shows photographs of anther morphology of each of an intergeneric hybrid plant having the same cytoplasm as that of the novel cytoplasmic male sterile line and an intergeneric hybrid plant having a normal cytoplasm taken with a stereomicroscope.

### [Description of Embodiments]

The present invention will be described in detail.

### Novel cytoplasmic male sterile plant of the genus Petunia, or hybrid plant with the cytoplasmic male sterile plant of the genus Petunia, or progeny thereof

The present invention as mentioned above relates to a cytoplasmic male sterile plant of the genus Petunia having, in the mitochondrial genome thereof, a DNA molecule originated from the mitochondrial genome of a tobacco plant, or a hybrid plant with the cytoplasmic male sterile plant of the genus Petunia, or a progeny of either the cytoplasmic male sterile plant of the genus Petunia or the hybrid plant. The cytoplasmic male sterile plant of the genus Petunia or the hybrid plant with the cytoplasmic male sterile plant of the genus Petunia, or the progeny thereof according to the present invention has the growth ability of young seedlings, which is improved as compared with a known cytoplasmic male sterile plant of the genus Petunia.

The cytoplasmic male sterile plant of the genus Petunia according to the present invention has, in the mitochondrial genome thereof, a DNA molecule originated from a mitochondrial genome of a tobacco plant.

The "tobacco plant", i.e., a Nicotiana plant can be used as a cytoplasmic donor parent in the present invention. The Nicotiana plant is preferably N. suaveolens, N. debneyi, N. acuminata, or N. longiflora, and more preferably Nicotiana suaveolens.

It is possible to confirm if the cytoplasmic male sterile plant of the genus Petunia according to the present invention has, in the mitochondrial genome thereof, a DNA molecule originated from a mitochondrial genome of a tobacco plant by, for example, the method using a predetermined primer described below as an index described in Examples of the present specificattion.

Examples of the "plant of the genus Petunia" used for making the cytoplasmic male sterile plant of the genus Petunia according to the present invention include P. hybrida, P. axillaris, P. integrifolia, P. alpicola, P. altiplana, P. bajeensis, P. bonjardinensis, P. exserta, P. guarapuavensis, P. helianthemoides, P. humifusa, P. inflata, P. interior, P. ledifolia, P. littoralis, P. mantiqueirensis, P. occidentalis, P. patagonica, P. pubescens, P. reitzii, P. riograndensis, P. saxicola, P. scheideana, P. variabilis, and P. villadiana. In addition, the "plant of the genus Petunia" can be originated from an interspecific hybrid plant of a species belonging to Petunia, and among them, the "plant of the genus Petunia" is preferably P. hybrida, which is a cultivated species of Petunia.

The term "interspecific hybrid plant" refers to a plant produced by interspecific hybridization, protoplast fusion, or grafting between different species in the species belonging to the plant of the genus Petunia as exemplified above.

Therefore, according to a preferred aspect of the present invention, the cytoplasmic male sterile plant of the genus Petunia is Petunia hybrida, or originated from an interspecific hybrid plant of a plant of the genus Petunia.

In the present description, the term "hybrid plant with a plant of the genus Petunia" means a plant originated from an intergeneric hybrid plant produced by crossing between a plant of the genus Petunia and a plant of a closely related genus. Examples of the plant of a closely related genus include plants of the genus Calibrachoa, the genus Nierembergia, and the genus Brunfelsia. A preferred closely related genus is the genus Calibrachoa or the genus Nierembergia, and more preferably the genus Calibrachoa.

Therefore, according to a preferred aspect of the present invention, the hybrid of the cytoplasmic male sterile plant of the genus Petunia is originated from an intergeneric hybrid plant of a plant of the genus Petunia and a Calibrachoa plant, and includes a plant produced by protoplast fusion or grafting.

The term "progeny of a cytoplasmic male sterile plant of the genus Petunia" as used herein means a cytoplasmic male sterile plant of the genus Petunia of the next generation that is produced by cross-pollinating a plant of the genus Petunia capable of being crossbred with a cytoplasmic male sterile plant of the genus Petunia and thereby acquiring the cytoplasm by cytoplasmic inheritance. Therefore, the progeny includes not only a progeny using a cytoplasmic male sterile plant of the genus Petunia having, in the mitochondrial genome thereof, a DNA molecule originated from a mitochondrial genome of a tobacco plant, but also a progeny using a hybrid plant with the cytoplasmic male sterile plant of the genus Petunia, and further includes a hybrid species produced by cross-pollinating the cytoplasmic male sterile plant of the genus Petunia or the hybrid plant thereof according to the present invention with a plant of the genus Petunia capable of being crossbred with the plant. Therefore, the term "progeny of a cytoplasmic male sterile plant of the genus Petunia" also includes, for example, a plant produced by cross-pollinating the cytoplasmic male sterile plant of the genus Petunia according to the present invention, which is used as a seed parent (i.e., a female parent), with a plant of the genus Petunia capable of being crossbred with the plant, which is used as a pollen parent (i.e., a male parent). In addition, the "progeny of a cytoplasmic male sterile plant of the genus Petunia" also includes, for example, a somatic cell hybrid plant produced by protoplast fusion between the cytoplasmic male sterile plant of the genus Petunia according to the present invention and the plant of the genus Petunia, or a grafted hybrid plant.

The term "asymmetric protoplast fusion" as used herein refers to a technique where a nuclear genome of one of isolated protoplasts that are to be used for protoplast fusion is disrupted prior to the fusion and then the protoplast fusion is carried out using the disrupted nuclear genome. In the asymmetric protoplast fusion, a protoplast in which a nuclear genome is disrupted prior to fusion and of which a cytoplasm is donated to a fused cell through protoplast fusion is referred to as a "cytoplasm donor parent". A protoplast in which a nuclear genome is maintained without being disrupted prior to fusion and which receives the cytoplasm from the cytoplasm donor parent upon the fusion is referred to as a "cytoplasm acceptor parent".

The term "asymmetric back protoplast fusion" means that one or more (preferably one) additional asymmetric protoplast fusions are carried out using the plant produced by the asymmetric protoplast fusion or a progeny thereof as a cytoplasm donor parent and using one of the plants used for the initial asymmetric protoplast fusion as a cytoplasm acceptor parent. That is, in asymmetric back protoplast fusion, asymmetric protoplast fusion is carried out two or more times, including the first time.

In the present invention, the term "normal cytoplasm" is typically used in the meaning that the cytoplasm does not exhibit sterility and is normal in contrast to a plant cytoplasm exhibiting male sterility, i.e., a male sterile cytoplasm.

The cytoplasmic male sterile plant of the genus Petunia of the present invention is obtained by preferably using a tobacco plant as a cytoplasm donor parent in asymmetric protoplast fusion. At this time, it is desirable to use a plant of the genus Petunia having a normal cytoplasm as a cytoplasm acceptor parent.

According to a preferred aspect of the present invention, in the cytoplasmic male sterile plant of the genus Petunia or the hybrid plant with the cytoplasmic male sterile plant of the genus Petunia, or the progeny thereof, at least one of mitochondrial DNA regions is a Nicotiana suaveolens type wherein the regions are identified by a mitochondrial genome marker using one or more primers selected from a primer set (primer No. 30) having nucleotide sequences set forth in SEQ ID NOs: 59 and 60 and a primer set (primer No. 34) having nucleotide sequences set forth in SEQ ID NOs: 67 and 68.

In other words, in the cytoplasmic male sterile plant of the genus Petunia or the hybrid plant with the cytoplasmic male sterile plant of the genus Petunia, or the progeny thereof, at least one of mitochondrial DNA regions identified by a mitochondrial genome marker using one or more primers selected from primers SNc324k-3F, SNc325k-4R, SNc382k-1F, and SNc383k-4R (primers having nucleotide sequences set forth in SEQ ID NOs: 59, 60, 67, and 68) is a Nicotiana suaveolens type.

According to a more preferred aspect of the present invention, the cytoplasmic male sterile plant of the genus Petunia or the hybrid plant with the cytoplasmic male sterile plant of the genus Petunia, or the progeny thereof according to the present invention includes a mitochondrial genome originated from a plant identified by Accession No. FERM BP-22398 (details will be explained below), and is more preferably identified by Accession No. FERM BP-22398.

The term "a part of a plant body" of the cytoplasmic male sterile plant of the genus Petunia or a hybrid plant with the cytoplasmic male sterile plant of the genus Petunia, or a progeny thereof as used herein includes one or more cells of the plant body or a cytoplasm composed of one or more cells of the plant body, and specifically refers to an organ (e.g., a flower, a leaf, a stem, a root) or a tissue, or a cell (including a protoplast prepared from cells) or a cytoplasm from the organ or the tissue, or a mass of the cells or the cytoplasms.

### Method for constructing novel cytoplasmic male sterile plant of the genus Petunia

The novel cytoplasmic male sterile plant of the genus Petunia according to the present invention can be constructed, for example, in accordance with the following procedure.
(1) Preparation of a protoplast.
(2) Asymmetric protoplast fusion.
(3) Culture of a fused hybrid cell.
(4) Selection of a cytoplasm hybrid plant having cytoplasmic male sterility.
(5) Regeneration of a plant body from a callus.
(6) Acquisition of a progeny and selection of a high-quality line.

In the description, the term "production method" can also be referred to as the term "construction method". The terms "construction" and "production" can be used interchangeably with each other.

These steps are more specifically explained as follows.
(1) Preparation of a protoplast.
   (i) Isolation of a protoplast of a plant of the genus Petunia.

Examples of the "plant of the genus Petunia" used for preparation of a protoplast include P. hybrida, P. axillaris, P. integrifolia, P. alpicola, P. altiplana, P. bajeensis, P. bonjardinensis, P. exserta, P. guarapuavensis, P. helianthemoides, P. humifusa, P. inflata, P. interior, P. ledifolia, P. littoralis, P. mantiqueirensis, P. occidentalis, P. patagonica, P. pubescens, P. reitzii, P. riograndensis, P. saxicola, P. scheideana, P. variabilis, and P. villadiana, and among them, P. hybrida, which is a cultivated species of petunia, is preferable.

For the cell tissue to be used for producing the protoplast, it is desirable to provide a mesophyll tissue that has a high yielding ability and a high division activity. Alternatively, another tissue, e.g., a hypocotyl, a stem and a callus, may also be used as a material for the cell tissue.

The method for isolating the protoplast may be a known method that has been used commonly in the art (e.g., the method described in Matsumoto, E, Plant cell reports, 1991. vol9 (10) or the like), and is not particularly limited. Specific examples of the procedure will be described below, but the present invention is not limited to these examples.

First, a tissue of a plant of the genus Petunia is finely cut and is then enzymatically treated using an enzyme solution for protoplast isolation use to isolate protoplasts. The solution is an inorganic salt buffer mainly containing a cell wall degradation enzyme and an osmotic pressure regulator. The cell wall degradation enzyme is not particularly limited, as long as the cell wall degradation enzyme can be used for the degradation of a cell wall of a plant. Examples the cell wall degradation enzyme include a cellulase, a hemicellulase and a pectinase. In the present invention, a combination of Cellulase Y-C and Macerozyme R-10 is preferred.

The osmotic pressure regulator preferably includes a common sugar alcohol, e.g., mannitol, sorbitol and glucose, more preferably mannitol, and most preferably mannitol at a concentration of 0.3 M to 0.7 M. Furthermore, it is desirable to add an inorganic salt to the enzyme solution for the purpose of stabilizing a membrane of the protoplast, and for example, to preferably add a CPW salt (Cocking and Peberdy, 1974) having the composition shown in Table 1. The enzymatic treatment is preferably carried out by statically treating at 25 to 30°C for 8 to 20 hours.

**[Table 1]**

| Composition of CPW salt solution | |
|---|---|
| KH₂PO₄ | 27.2 mg/l |
| KNO₃ | 101.0 mg/l |
| CaCl₂.2H₂O | 1,480.0 mg/l |
| MgSO₄ | 246.0 mg/l |
| KI | 0.16 mg/l |
| CuSO₄.5H₂O | 0.025 mg/l |
| Mannitol | 0.6 M |
| pH | 5.8 |

The protoplasts isolated by the enzymatic treatment are filtrated through a nylon mesh having a pore diameter of 30 to 100 µm, then the filtrate is centrifuged, the protoplasts are collected and the enzyme solution is discarded. Next, the protoplasts are suspended in a wash solution to wash the protoplasts. The wash solution used can be one prepared by adding a sugar alcohol as an osmotic pressure regulator to a commonly used CPW salt solution.

Next, it is desirable to carry out an inactivation treatment for the purpose of preventing the division of the plant of the genus Petunia protoplasts alone. The inactivation treatment can be carried out by suspending the protoplast in a CPW salt solution having an iodo compound, e.g., iodoacetic acid and iodoacetamide, dissolved therein. In the present invention, it is preferred to suspend the protoplast in a CPW salt solution containing iodoacetamide at a concentration of 5 mM to 30 mM and then carry out the treatment for 5 to 20 minutes.

Next, it is preferred to repeat the washing step with a CPW salt solution using a centrifuge machine 1 to 3 times. The suspension of protoplasts will be contaminated with vessels and cell fragments. Therefore, it is preferred to further purify the suspension by a density gradient centrifugation method or the like.

Examples of the reagent to be used for the purification include a sugar and a synthetic colloid. In the present invention, it is preferred to use a sucrose solution, and it is particularly preferred to use a sucrose solution having a concentration of 15% to 20%. After the purification of the protoplasts, the cell density is measured using a hemocytometer, and the volume of the solution is adjusted with a CPW salt solution in such a manner that the cell density can have a value suitable for protoplast fusion. The cell density of the protoplast suspension is preferably 1 × 10⁵ to 1×10⁷ cells/ml, and a CPW salt solution is preferably used for the adjustment of the volume of the solution.

### (ii) Isolation of a protoplast of a Nicotiana plant.

A Nicotiana plant can be used as the cytoplasm donor parent of the present invention. Among the Nicotiana plants, N. suaveolens, N. debneyi, N. acuminata, and N. longiflora are preferable, and N. suaveolens, which has an example of construction of a cytoplasmic male sterile line of tobacco (N. tabacum), is particularly preferable.

The isolation of protoplasts of the Nicotiana plant can be carried out in accordance with, for example, the same method as the above method employed for the isolation of protoplasts of the plant of the genus Petunia.

It is desirable to inactivate the nucleus of the isolated protoplast of the Nicotiana plant by a radioactive ray treatment before using. Examples of the ray to be emitted for the radioactive ray treatment include X-ray, γ -ray, and ultraviolet ray. The radioactive ray is not particularly limited, as long as the nucleus can be destroyed. The exposure radiation dose is preferably as small as possible within such a range that the nucleus can be destroyed. For example, in the present invention, in the case of the irradiation with soft X-ray, the exposure radiation dose is preferably 100 Gy to 900 Gy.

### (2) Fusion treatment of the protoplasts.

Second, the protoplasts of both species obtained in the above steps are combined to carry out protoplast fusion.

Examples of the fusion method include, but are not particularly limited to, conventional methods, such as a known electrical fusion method (Planta, 151, 26-32, 1981), a PEG (polyethylene glycol) method (Planta, 120, 215-227, 1974) and a dextran method (Jap. J. Genet., 50, 235, 1975). In the present invention, it is preferred to employ a PEG method.

### (3) Culture of a fused hybrid cell.

The cell obtained by the fusion treatment is preferably cultured in a culture medium that is suitable for the culture of a protoplast from a plant of the genus Petunia. The method for culturing a protoplast of a plant of the genus Petunia is not particularly limited, and is appropriately modified on the basis of a method for culturing a protoplast of a Petunia plant. In the present invention, it is preferred to use a half-strength MS medium in which the concentration of NH₄NO₃ is reduced to 200 mg/l (Murashige, T. & Skoog, Physiol. Plant., 15, 473-497, 1962) as a basal medium and is appropriately supplemented with a plant growth regulating substance, various additives and the like upon use.

### (4) Selection of a cytoplasm hybrid plant having cytoplasmic male sterility.

The fused protoplasts are cultured. At the stage where cell division starts and a callus can be visually confirmed, the callus is transplanted into a callus proliferation medium. A conventional callus proliferation medium can be used. For example, an MS medium containing 0.1 to 3.0 mg/l naphthaleneacetic acid (NAA) and 0.1 to 3.0 mg/l thidiazuron (TDZ) can preferably be used, although success may vary depending on the genotype of the plant material that is used or the condition of the callus.

In the selection of a cytoplasmic hybrid individual, it is preferable to extract DNA from the callus and efficiently select the individual using a marker capable of specifically amplifying the mitochondrial DNA of a Nicotiana plant by a PCR method.

### (5) Regeneration of a plant body from a callus.

The callus having the mitochondrial DNA of the selected Nicotiana plant is transplanted into a regeneration medium and regenerated.

A conventional regeneration medium can be used. For example, an MS medium supplemented with 0.1 to 1.0 mg/l NAA and 0.1 to 1.0 mg/l TDZ can preferably be used, although success may vary depending on the genotype of the plant material used or the condition of the callus.

A regenerated shoot is transplanted into an MS medium supplemented with 3% sucrose and 0.8% agar to cause the rooting of the shoot, thereby regenerating a plant. The regenerated plant body is acclimated and is then grown in a greenhouse.

### (6) Acquisition of a progeny and selection of a high-quality line.

In the cytoplasm hybrid plant thus obtained, protoplasts of two or more lants of the genus Petunia may be fused to form a higher polyploid such as a tetraploid or higher. Since the higher polyploid has poor progeny acquisition efficiency and it takes time and labor to return to a diploid, it is preferable to test ploidy using a flow cytometer and use only a diploid individual. The cytoplasm hybrid plant thus obtained is grown in a greenhouse or the like and flowered, and an individual exhibiting male sterility is selected. A plant of the genus Petunia having a normal cytoplasm is crossbred as a pollen parent, and it is confirmed that the progeny has male sterility.

When heterologous cells are fused by protoplast fusion, their mitochondria repeat fusion and division, resulting in recombination between heterologous mitochondrial genomes. In plants, since there are several hundreds to several tens of thousands of mitochondria in one cell and each recombines, the cytoplasm hybrid plant becomes a heteroplasmy state immediately after protoplast fusion. In order to eliminate the heteroplasmy state and to evaluate the stability of cytoplasmic male sterility and other traits, it is desirable to carry out recurrent backcrossing seven or more times.

### Method for improving novel cytoplasmic male sterile plant of the genus Petunia

If the cytoplasmic male sterile line thus obtained was associated with a deleterious trait, the trait can be improved by asymmetric back protoplast fusion. Asymmetric back protoplast fusion is a method of improving a mitochondrial genome by carrying out asymmetric protoplast fusion using a plant produced by asymmetric protoplast fusion or a progeny thereof as a cytoplasm donor parent and using a plant having a normal cytoplasm as a cytoplasm acceptor parent.

The causative gene of cytoplasmic male sterility is generally present in a mitochondrial genome. The novel Petunia cytoplasmic male sterile line produced by the first asymmetric protoplast fusion is believed to also have the mitochondrial DNA of N. suaveolens that causes expression of deleterious traits not involved in male sterility, in addition to the mitochondrial DNA of N. suaveolens that induces male sterility.

Asymmetric back protoplast fusion, i.e., a second asymmetric protoplast fusion can be carried out by using a novel Petunia cytoplasmic male sterile line produced by the first asymmetric protoplast fusion as a cytoplasm donor parent and using a plant of the genus Petunia having a normal cytoplasm as a cytoplasm acceptor parent, to generate a new recombination of the mitochondrial genome. At this time, by using a novel Petunia cytoplasmic male sterile line produced by the first asymmetric protoplast fusion in which the entire mitochondrial genome is close to a petunia type as a material, a mitochondrial genome closer to the petunia type is easily obtained, and selection of a high-quality cytoplasmic male sterile line becomes efficient.

Similar to the first asymmetric protoplast fusion, the cytoplasm hybrid plant thus obtained is in a heteroplasmy state immediately after the protoplast fusion, and thus it is necessary to select a high-quality cytoplasmic male sterile line while carrying out recurrent backcrossing. It is also possible to further improve the mitochondrial genome by repeating asymmetric back protoplast fusions two or more times.

### [Examples]

The present invention will be described more specifically with reference to the following examples. However, the present invention is not limited to these examples.

### Example 1: Method for constructing novel Petunia cytoplasmic male sterile line

(1) Preparation of a protoplast.
   (i) Isolation of a protoplast of a plant of the genus Petunia having a normal cytoplasm.

For a plant of the genus Petunia having a normal cytoplasm, "Pt1" having a reddish-purple flower color and being a grandiflora type was used. Sterilized seeds of "Pt1" were bedded on MS medium supplemented with 3% sucrose and 0.8% agar, and then grown for about 1 month at 20°C under 16-hour lighting. Opened true leaves (about 1 g) were collected, finely cut to a width of about 2 mm, immersed in a CPW salt solution (10 ml) containing 0.3% Cellulase Y-C, 0.3% Macerozyme R-10 and 0.5 M mannitol, and then allowed to incubate at 25°C for 16 hours.

The enzyme solution containing leaf tissues was filtrated through a 59-µm nylon mesh to remove cell debris. A protoplast suspension thus obtained was transferred to a centrifuge tube and centrifuged at 800 rpm for 5 minutes. Protoplasts isolated by discarding the supernatant were suspended in a CPW salt solution (5 ml) containing 15 mM iodoacetamide were then incubated at 4°C for 15 minutes. After the incubation, the protoplast suspension which had been treated with iodoacetamide was centrifuged at 800 rpm for 5 minutes, and then the supernatant was discarded. A procedure including adding a CPW salt solution (10 ml) to the protoplast suspension, then carrying out centrifugation at 800 rpm for 5 minutes and then discarding the supernatant was repeated three times to wash the protoplasts.

The washed protoplast suspension was centrifuged at 800 rpm for 5 minutes, the supernatant was discarded from the suspension and then a CPW salt solution (2 ml) was added to the resultant solution to suspend the protoplasts. A CPW salt solution (5 ml) supplemented with 20% sucrose was added to a new centrifuge tube, the protoplast suspension was overlaid over the CPW salt solution, and the resultant solution was centrifuged at 800 rpm for 5 minutes. The cell debris settled to the bottom of the centrifuge tube and the purified protoplasts floated in an upper layer of the CPW salt solution. Then the purified protoplasts were transferred to a new centrifuge tube with a Pasteur pipette. A small portion of the suspension was removed, the cell density of the protoplasts was determined using a hemocytometer and then a CPW solution was added to the suspension in such a manner that the density of the protoplasts became 1×10⁶ cells/ml.

### (ii) Isolation of a protoplast of a Nicotiana plant.

For a Nicotiana plant, N. suaveolens, which is used to be an example of construction of a cytoplasmic male sterile plant of tobacco, was used. Tobacco seeds of N. suaveolens were provided by Leaf Tobacco Research Laboratory, Japan Tobacco Inc.

Sterilized N. suaveolens seeds were bedded on MS medium supplemented with 3% sucrose and 0.8% agar, and then grown for about 1 month at 20°C under 16-hour lighting. Opened true leaves (about 1 g) were collected, finely cut into sizes of about 2 mm, immersed in a CPW salt solution (10 ml) containing 0.3% Cellulase Y-C, 0.3% Macerozyme R-10 and 0.5 M mannitol, and then allowed to incubate at 25°C for 16 hours.

The enzyme solution containing leaf tissues was filtrated through a 59-µm nylon mesh to remove cell debris. Protoplasts were transferred onto a plastic petri dish with a Pasteur pipette, and then irradiated with 900 Gy of soft X-ray.

The resultant protoplast suspension was transferred into a centrifuge tube, centrifuged at 800 rpm for 5 minutes, the supernatant was discarded from the suspension and then a CPW salt solution (2 ml) was added to the resultant solution to suspend the protoplasts. A CPW salt solution (5 ml) supplemented with 20% sucrose was added to a new centrifuge tube, the protoplast suspension was overlaid over the CPW salt solution and the resultant solution was centrifuged at 800 rpm for 5 minutes. The cell debris settled to the bottom of the centrifuge tube and the purified protoplasts floated in an upper layer of the CPW salt solution. Then the purified protoplasts were transferred to a new centrifuge tube with a Pasteur pipette. A small portion of the suspension was removed, then the cell density of the protoplasts was determined using a hemocytometer and then a CPW salt solution was added to the suspension in such a manner that the density of the protoplasts became 1×10⁶ cells/ml.

### (2) Fusion treatment of the protoplasts.

The protoplast suspension of a plant of the genus Petunia having a normal cytoplasm which had been treated with iodoacetamide and the Nicotiana plant protoplast suspension which had been irradiated with soft X-ray were combined at a ratio of 1:3, and the resultant solution (2 ml) was dropped onto the bottom center of a 9-cm petri dish. The mixed solution was allowed to incubate for 30 minutes, and then 3 ml of a PEG solution (500 g/l polyethylene glycol #6000 (nacalai tesque Inc.), 1,500 mg/l of CaCl₂·2H₂O, 100 mg/l of KH₂PO₄, pH 5.5) was dropped around the protoplast solution.

After 1 minute, a CPW salt solution (3.5 ml) was dropped around the protoplast solution. After an additional 2 minutes, additional CPW salt solution (3.5 ml) was dropped around the protoplast solution. After 5 minutes, the dropped solution was removed by gently drawing up from the edge of the petri dish and a CPW salt solution (20 ml) was added from the edge of the petri dish. The washing procedure with the CPW salt solution was repeated 3 times at 5-minute intervals.

### (3) Culture of a fused hybrid cell.

After the removal of the wash solution, a half-strength MS medium (10 ml) (pH 5.8) which contained 0.5 M mannitol, 150 mg/l casamino acid, 100 mg/l L-glutamine, 0.1 mg/l NAA, 0.1 mg/l 2,4-D (2,4-dichlorophenoxyacetic acid), 0.1 mg/l TDZ and 1% sucrose, and in which the concentration of NH₄NO₃ was reduced to 200 mg/l, was added to the solution, and the resultant solution was cultured at 25°C in a dark place.

Seven days after the culture started, a half-strength MS medium (5 ml) (pH 5.8) which contained 150 mg/l casamino acid, 100 mg/l L-glutamine, 0.1 mg/l NAA, 0.1 mg/l 2,4-D, 0.1 mg/l BA and 1% sucrose, and in which the concentration of NH₄NO₃ was reduced to 200 mg/l, was added to reduce the concentration of mannitol, and then the culture was continued.

Ten days after the culture started, cells adhering onto the bottom of the petri dish were detached by rubbing with the tips of tweezers, a solution (7.5 ml) containing 0.2 M mannitol, 4% sucrose and 0.6% gellan gum was added to and mixed with the cells to form a half-solidified gel medium, and then the culture was continued.

About 1 month after the culture started, a callus could be identified by eye. At such time the callus was transplanted onto a callus proliferation medium (an MS medium containing 1 mg/l NAA, 1 mg/l TDZ, 3.0% sucrose and 0.8% agar, pH 5.8).

### (4) Selection of a cytoplasm hybrid plant having cytoplasmic male sterility.

To select a cytoplasm hybrid plant, molecular markers capable of detecting DNA specific to N. suaveolens by a PCR test were designed. Since the nucleotide sequence of the mitochondrial genome of N. suaveolens is not available, the nucleotide sequence information of N. tabacum of the same Tobacco genus (GenBank Accession No. BA000042) was used to design primers specific to the nad3 gene (Table 2).

At the stage where the callus grew to 5 mm or larger, a part of the callus was sampled and DNA was extracted therefrom. PCR was carried out using the extracted full-length genome DNA as a template and using a combination of primer No. 1. In the PCR, denaturation at 94°C for 1 minute, annealing at 60°C for 2 minutes and an extension reaction at 72°C for 2 minutes were repeated for 35 cycles.

A PCR product was electrophoresed on a 1.8% agarose gel, and the gel was immersed in an ethidium bromide solution and then photographed under irradiation with UV light. Individuals having a band corresponding to an expected size (456 bp) were selected.

### [Table 2]

**Table 2: Nucleotide sequence information of primers used in selection of cytoplasmic hybrids**

| Primer No. | Primer name | Nucleotide sequence (5' → 3') | SEQ ID NO |
|---|---|---|---|
| 1 | PN-Nad3-3F | GCTCAAGCGCATCATTTTCGTG | Seq ID-1 |
| | PN-Nad3-2R | CATCACCGGAAGGATCGAAACC | Seq ID-2 |

### (5) Regeneration of a plant body from a callus.

At the stage where the callus grew to about 1 cm, the callus was cut into pieces about 2 mm and these were transplanted onto a regeneration medium (an MS medium containing 0.1 mg/l NAA, 1.0 mg/l TDZ, 3.0% sucrose and 0.8% agar, pH 5.8).

The shoot regeneration of the callus started about 1 month after transplanting onto the regeneration medium. The regenerated shoots were rooted by transplanting onto an MS medium (pH 5.8) containing 3.0% sucrose and 0.8% agar. Each of the cytoplasm hybrid plants was transplanted and acclimated in a 72-hole cell tray. The cytoplasm hybrid plants were examined with respect to polyploidy using a flow cytometer. The cytoplasm hybrid plants included diploid and tetraploid, and the occurrence of aneuploids was not observed.

### (6) Acquisition of a progeny and selection of a high-quality line.

Each of the cytoplasm hybrid plants was transplanted into a 9-cm pot to continue seedling-raising, and male sterility was examined after flowering. As a result, 12 male sterile lines were obtained.

Recurrent backcrossing was carried out using 12 lines exhibiting male sterility as a seed parent (nonrecurrent parent) and a multiflora petunia parent line "Pt2" having a normal cytoplasm and pink flower color as a pollen parent (recurrent parent).

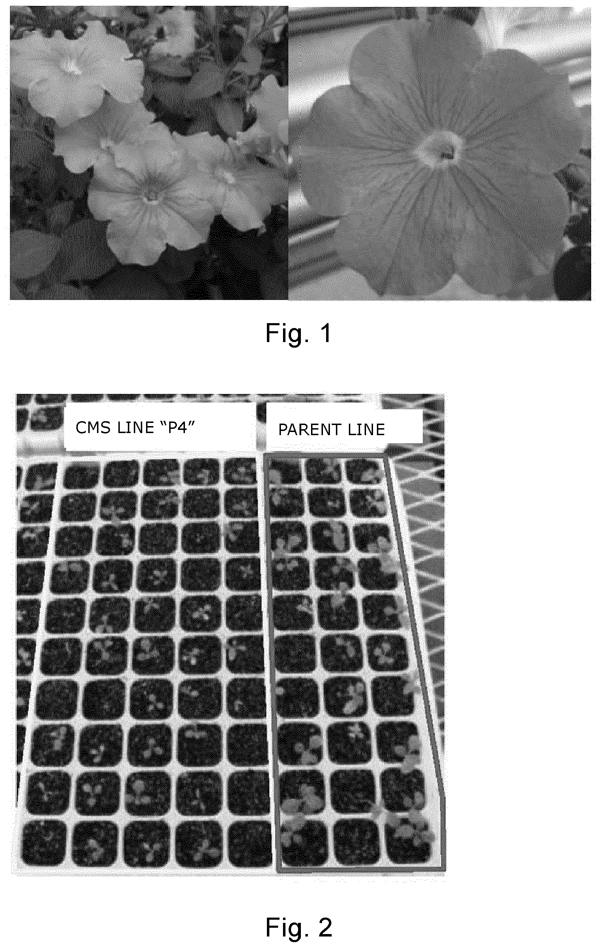

Seven recurrent backcrossings were carried out to select a novel Petunia cytoplasmic male sterile line "P4" that had stable cytoplasmic male sterility, high female fertility and exhibited normal morphology (Fig. 1).

The anthers of "P4" were completely degenerated and necrotic, and no pollen was produced. "P4" had been subjected to seven recurrent backcrossings of "Pt2", and the mitochondrial genome was in homoplasmy and the cytoplasm was considered stable.

Next, in order to use "P4" as a seed parent for F1 variety development, 32 lines of various fertile parent lines (self-fertilized lines) were recurrently backcrossed to make male sterile parent lines.

As a result, in the recurrently backcrossed progeny of most parent lines, deterioration in the growth ability of young seedlings was observed, and it was found that, in practice, there was a problem. Fig. 2 shows a remarkable example in which deterioration in the growth ability of a young seedling was observed.

As shown in Fig. 2, it was confirmed that in the recurrently backcrossed progeny (BC3) of the novel Petunia cytoplasmic male sterile line "P4", the growth ability of young seedlings was deteriorated as compared with its recurrent parent line.

In the seventh generation (BC7) of recurrent backcrossing of "Pt2", deterioration in the growth ability of a young seedling was not observed, and thus it was considered that deterioration in the growth ability (incompatibility between nucleus and cytoplasm) was caused depending on the genotype of the nuclear genome of the breeding parent line. As described above, in the cytoplasmic male sterile line "P4", it was considered that the growth ability at the time of young seedling is deteriorated in the recurrently backcrossed progeny of most parent lines, and it was considered that improvement of the mitochondrial genome is necessary for breeding utilization.

### Example 2: Method of improving novel petunia cytoplasmic male sterile line "P4"

In order to improve the mitochondrial genome of the novel petunia cytoplasmic male sterile line "P4" produced by the first asymmetric protoplast fusion, asymmetric protoplast fusion (asymmetric back protoplast fusion) was carried out in the same manner as in Example 1 using "P4" produced by the first asymmetric protoplast fusion as a cytoplasm donor parent and using a parent line "Pt3", which is a plant of the genus Petunia having a normal cytoplasm and is a multiflora type having a red flower color, as a cytoplasm acceptor parent.

Since "Pt3" is a line in which deterioration in the growth ability of a young seedling is observed when "Pt3" is recurrently backcrossed with "P4", it is possible to select a target cytoplasmic male sterile line by comparing the growth ability at a young seedling stage of a line produced by asymmetric back protoplast fusion.

For the callus thus obtained, cytoplasmic hybrids were selected using primers specific to the tobacco nad3 gene as in Example 1.

Each of the cytoplasm hybrid plants was transplanted into a 9-cm pot to continue seedling-raising, and male sterility was examined after flowering. As a result, 14 cytoplasmic male sterile lines were obtained.

Recurrent backcrossing was carried out using 14 lines exhibiting male sterility as a seed parent (nonrecurrent parent) and a multiflora plant of the genus Petunia "Pt3" having a normal cytoplasm and a red flower color (the same as the cytoplasm acceptor parent) as a pollen parent (recurrent parent).

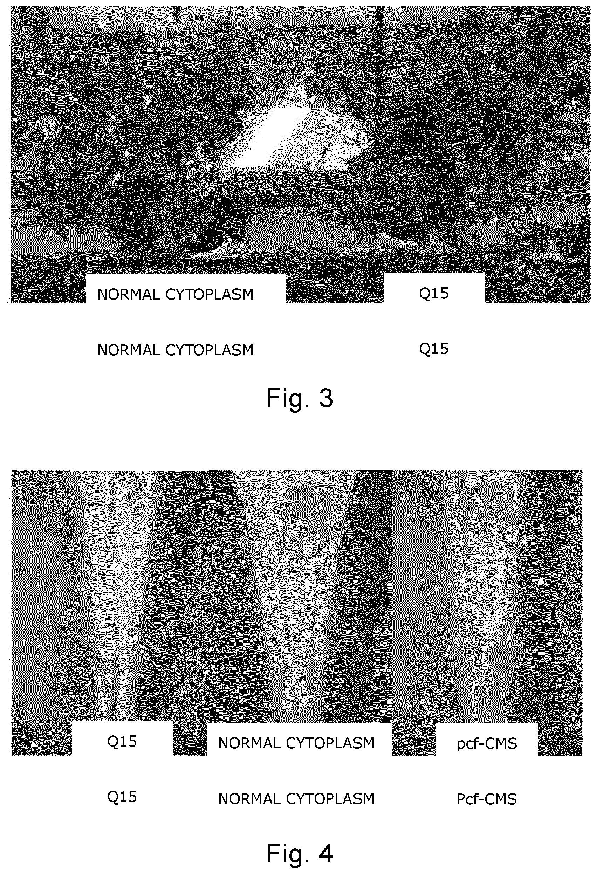

Seven recurrent backcrossings were carried out to select a novel Petunia cytoplasmic male sterile line "Q15" that had stable cytoplasmic male sterility, high female fertility and exhibited normal morphology (Fig. 3).

Fig. 4 shows anther morphology of each of "Pt3" having a normal cytoplasm, a cytoplasmic male sterile line "pcf-CMS" produced by recurrent backcrossing (BC7) of "Pt3", and "Q15".

"pcf-CMS" was found to have degenerated anthers and to produce non-fertile pollen grains, while "Q15" was found to have fully degenerated anthers and to produce no pollen. The cytoplasmic male sterile line "Q15" had been subjected to seven backcrossings, and the mitochondrial genome was in homoplasmy and the cytoplasm was considered stable.

"Q15" had been confirmed to have the tobacco nad3 gene at the time of the callus obtained upon asymmetric back protoplast fusion, whereas the tobacco nad3 gene had disappeared in the BC7 generation that had been subjected to seven backcrossings. This was thought to be because the repeated selection was based on the growth ability at young seedling stage, while confirming that male sterility was exhibited in each generation of backcrossing, and thus the selection proceeded in a direction in which the mitochondrial gene from N. suaveolens, which is considered to be a cause of incompatibility between nuclear and cytoplasmic genomes of Petunia, was eliminated.

Therefore, in "Q15", since the tobacco nad3 gene used as a marker for cytoplasmic selection had disappeared, it was necessary to design new molecular markers and analyze the cytoplasm in order to confirm that the mitochondrial DNA of N. suaveolens was introduced into "Q15".

Since the nucleotide sequence information of the mitochondrial genome of N. suaveolens is not available, the entire nucleotide sequence information of N. tabacum of the same tobacco genus (GenBank Accession No. BA000042) was used to design primers that specifically amplify 16 mitochondrial genes, and the primers are shown as primer Nos. 2 to 17 in Table 3.

In addition, primers that specifically amplify the pcf gene were designed based on the nucleotide sequence information of the CMS causative gene (pcf) of known Petunia, and are shown as primer No. 18 in Table 3.

Furthermore, to identify the origin of the chloroplast of "Q15", primers that specifically amplify the spacer region between the atpA gene and the atpH gene were designed using the entire nucleotide sequence information of the chloroplast genome of N. tabacum (GenBank Accession No. Z00044), and these are shown as primer No. 19 in Table 3. The PCR product was digested with a restriction enzyme TaqI, and RFLP due to a difference in restriction enzyme site was detected to identify the origin of the chloroplast.

### [Table 3]

**Table 3: Nucleotide sequence information of primers designed based on nucleotide sequence information of known plant of the genus Petunia and N. tabacum**

| Primer No. | Primer name | Nucleotide sequence (5' → 3') | SEQ ID NO |
|---|---|---|---|
| 2 | SNtAtp6-1F | AAGCTCTACCAAGGATTGCCA | Seq ID-3 |
| | SNtAtp6-2R | GCTGCTTTCGGGTGATTGGA | Seq ID-4 |
| 3 | SNtNad1ex5-1F | CGCCAACTCCTATCCCGAAG | Seq ID-5 |
| | SNtNad1ex5-2R | CGTCGAAGCTACCGCGTAT | Seq ID-6 |
| 4 | SNtRrn26-1F | CTGGGGTTGAAGAAGGTCCC | Seq ID-7 |
| | SNtRrn26-2R | TTCCAGTCTTCGAGGCAAGC | Seq ID-8 |
| 5 | SNtNad2ex1-1F | ATCGGGAATTATGGGCTCCG | Seq ID-9 |
| | SNtNad2exl-2R | AGTAATGTGGGTTGGCTTGGA | Seq ID-10 |
| 6 | SNtRps12-1F | ATGGTGCGGAAAAACCCAA | Seq ID-11 |
| | SNtRps12-2R | ATGATTTCTCGCAACAGAATTAAGG | Seq ID-12 |
| 7 | SNtAtp4-1F | CTACACCGGGGGCAAAATCT | Seq ID-13 |
| | SNtAtp4-2R | GCGAGCGCATCCGATTCTAA | Seq ID-14 |
| 8 | SNtNad4L-1F | CGTCTATTAAGGAGGATTCCCCG | Seq ID-15 |
| | SNtNad4L-2R | GTCAGGCCCACCTTTCACAA | Seq ID-16 |
| 9 | SNtRps10-1F | AGGCAATCCAATCTTCCGTGT | Seq ID-17 |
| | SNtRps10-2R | GAAGCGATTGCAGCGAAGAA | Seq ID-18 |
| 10 | SNtAtp9-1F | CTTCGGTTAGAGCAAAGCCCA | Seq ID-19 |
| | SNtAtp9-2R | GCGTCTGTTCACGGAGGTT | Seq ID-20 |
| 11 | SNtRps19-1F | TACAATGGGAGACGAAGGCG | Seq ID-21 |
| | SNtRps19-2R | TTCGTACGGAGCAATCAACGA | Seq ID-22 |
| 12 | SNtCox2ex1-1F | ACAGGGAAGGTGGAAGAAATGA | Seq ID-23 |
| | SNtCox2ex1-2R | AAATGCCATAAAGCGCGACC | Seq ID-24 |
| 13 | SNtNad5ex4-1F | ATAGTGGGGGCCGAATCAAC | Seq ID-25 |
| | SNtNad5ex4-2R | TTGGAGCAGCAAACTCGGAT | Seq ID-26 |
| 14 | SNtOrfB-1F | TAAGGGGGTGTTCGAGTTGG | Seq ID-27 |
| | SNtOrfB-2R | CCATTCCTCGTGAGCCACTT | Seq ID-28 |
| 15 | SNtCox3-1F | GGGGTGCAACACTTCTCAGT | Seq ID-29 |
| | SNtCox3-2R | CCAGTTACCCGGAGTTCAAGT | Seq ID-30 |
| 16 | SNtAtp1-1F | GTAGGTGATGGGGCATAGCG | Seq ID-31 |
| | SNtAtp1-2R | CCAACTGAGACCACTCGACC | Seq ID-32 |
| 17 | SNtRpl2-1F | CCTTGGCCTTTGGAGAGGAG | Seq ID-33 |
| | SNtRpl2-2R | TTCCCTCTATCAGGAACCCGC | Seq ID-34 |
| 18 | SPhMt-PCF-1F | CTGCTATCGGTATTGGAAACG | Seq ID-35 |
| | SPhMt-PCF-2R | ATCGTGGCTTTCAGGTTTTG | Seq ID-36 |
| 19 | SNtCpAtpA-1F | CTTTCGATTCATTTGGCTCTC | Seq ID-37 |
| | SNtCpAtpA-2R | TTCCGTTATTGCTGCTGGATT | Seq ID-38 |

The results of analyzing the cytoplasm of each line of N. suaveolens, "Pt3", "P4", "Q15", and "pcf-CMS" using the newly designed primer sets in Table 3 are shown in Table 4.

### [Table 4]

**Table 4: Results of analyzing cytoplasm using primers designed based on nucleotide sequence information of known plant of the genus Petunia and N. tabacum**

| Primer No. | Target gene | Target region in N. tabacum (Genbank Accession No. BA000042) | D1 | D2 | D3 | D4 | D5 |
|---|---|---|---|---|---|---|---|
| 2 | atp6 | 25188 - 26260 | N | 0 | 0 | 0 | 0 |
| 3 | nad1 | 44305 - 45377 | N | 0 | 0 | 0 | 0 |
| 4 | rrn26 | 69908 - 70338 | N | 0 | 0 | 0 | 0 |
| 5 | nad2 | 75055 - 76501 | N | 0 | 0 | 0 | 0 |
| 6 | rps12 | 87149 - 88227 | N | P | P | P | P |
| 7 | Atp4 | 113950 - 114534 | N | 0 | 0 | 0 | 0 |
| 8 | nad4L | 114864 - 116042 | N | 0 | 0 | 0 | 0 |
| 9 | rps10 | 131486 - 132572 | N | 0 | 0 | 0 | 0 |
| 10 | atp9 | 145820 - 146205 | N | 0 | 0 | 0 | 0 |
| 11 | rps19 | 151578 - 152359 | N | 0 | 0 | 0 | 0 |
| 12 | cox2 | 156277 - 156542 | N | 0 | 0 | 0 | 0 |
| 13 | nad5 | 171627 - 172797 | N | 0 | 0 | 0 | 0 |
| 14 | orfB | 257506 - 258085 | N | 0 | 0 | 0 | 0 |
| 15 | cox3 | 258965 - 259975 | N | 0 | 0 | 0 | 0 |
| 16 | atp1 | 260046 - 261396 | N | 0 | 0 | 0 | 0 |
| 17 | rp12 | 363423 - 364258 | N | 0 | 0 | 0 | 0 |
| 18 | pcf | - | 0 | 0 | 0 | 0 | 1 |
| 19 | atpA(cp) | - | N | P | P | P | P |

| | | | | | | | |
|---|---|---|---|---|---|---|---|
| (Explanation of symbols in the table) D1: N. suaveolens D2: Pt3 (normal cytoplasm) D3: P4 D4: Q15 D5: pcf-CMS N: N. suaveolens type P: P. hybrida type 1: Detected with the marker 0: Not detected with the marker | | | | | | | |

Primers Nos. 2 to 17 designed this time were confirmed to cause specific amplification in N. suaveolens. It was confirmed that specific amplification did not occur in petunia having a normal cytoplasm except for primer No. 6, and that petunia-specific amplification occurred in primer No. 6.

For both "P4" and "Q15", specific amplification of an N. suaveolens type was not observed in all of the 16 primer sets designed this time. That is, it was suggested that in "P4" and "Q15", most of the mitochondrial genome had already been recombined into a Petunia type. "pcf-CMS" is believed to have originated from a wild species of the genus Petunia and no specific amplification of an N. suaveolens type was observed.

In addition, for "pcf-CMS", specific amplification could be confirmed by PCR using primer No. 18 that amplifies the pcf gene.

However, no specific amplification was observed in N. suaveolens, "P4", and "Q15", and it was confirmed that the pcf gene was not present in N. suaveolens, "P4", or "Q15", and "P4" and "Q15" exhibited cytoplasmic male sterility by a factor different from the pcf gene.

Analysis by PCR-RFLP of the chloroplast gene atpA by primer No. 19 showed that the chloroplasts of "P4" and "Q15" were of a Petunia type.

Since it has been found that chloroplast recombination basically does not occur in protoplast fusion, "P4" and "Q15" could be confirmed to have chloroplasts originated from petunia.

From the above results, it was confirmed that "P4" and "Q15" exhibit CMS by a mechanism different from the known pcf gene and have chloroplasts originated from petunia, and that main mitochondrial genes are recombined into a petunia type.

In tobacco (N. tabacum), "sua-CMS" in which the cytoplasm of N. suaveolens is introduced into N. tabacum by protoplast fusion is used, and the nucleotide sequence information of the mitochondrial genome (recombinant mitochondrial genome of N. tabacum and N. suaveolens) of "ms zhongyan100', which is a CMS line thereof, (GenBank Accession No. KR071121) is disclosed.

In addition, the nucleotide sequence information of the mitochondrial genome (N. tabacum) of "zhongyan100" having a normal cytoplasm and having the same nuclear genome (GenBank Accession No. KR780036) is also disclosed.

Therefore, by comparing the mitochondrial genomes of "ms zhongyan100" and "zhongyan 100" using Basic Local Alignment Search Tool (BLAST), regions specific to the mitochondrial genome of N. suaveolens were presumed, and primers that specifically amplify DNA of N. suaveolens were designed (Table 5).

### [Table 5]

**Table 5: Nucleotide sequence information of primers designed based on nucleotide sequence information of mitochondrial genome of "ms zhongyan100" (GenBank Accession No. KR071121)**

| Primer No. | Primer name | Nucleotide sequence (5' → 3') | SEQ ID NO |
|---|---|---|---|
| 20 | SNc13k-1F | TTTCGCCCTTGCTGCTGTAG | Seq ID-39 |
| | SNc13k-2R | TTCACTTCCAGTTAGGCAGGA | Seq ID-40 |
| 21 | SNc41k-1F | CCCTTGTTAGCCGAGCTGT | Seq ID-41 |
| | SNc41k-2R | AGGCACGGCCATACATAAGA | Seq ID-42 |
| 22 | SNc60k-1F | AGCCGAGAAGGAAGGGATGA | Seq ID-43 |
| | SNc60k-2R | TCGGTCCACCCTCTAGCTTT | Seq ID-44 |
| 23 | SNcOrf76-1F | CGTCACTGGCCTCCCATAAG | Seq ID-45 |
| | SNcOrf76-2R | TGCCTCTACTCCGCCTACTT | Seq ID-46 |
| 24 | SNc77k-1F | ACACAGCATACAGGAGGACG | Seq ID-47 |
| | SNc77k-2R | TATGTGGCAGCGGGAAAGAT | Seq ID-48 |
| 25 | SNc122k-1F | TCAGCCTTCCTTATCGTGGC | Seq ID-49 |
| | SNc122k-2R | AGTGCAGGAAAGGCAGAAGT | Seq ID-50 |
| 26 | SNcOrf115a-1F | AGGTCGGCCACAACTCCTAT | Seq ID-51 |
| | SNcOrf115a-2R | TGCTAAATCTGAGGGTGGCAT | Seq ID-52 |
| 27 | NcOrf82-1F | GCAGAGGTGACAAAGCAT | Seq ID-53 |
| | NcOrf82-2R | GAAACCAAACAAAGAGCG | Seq ID-54 |
| 28 | SNcOrf142-1F | GTGGCGTATGTCTACAGCGA | Seq ID-55 |
| | SNcOrf142-2R | GGAGCCCTGTTCGAGTGAAA | Seq ID-56 |
| 29 | SNcOrf73-1F | ACCTTTGCTTCGCCCCTATT | Seq ID-57 |
| | SNcOrf73-2R | TAGCACTGCTGCCACTTCTG | Seq ID-58 |
| 30 | SNc324k-3F | ACGTACCTCTCCCGTAGGTC | Seq ID-59 |
| | SNc325k-4R | CTGTGAAACGAAGCGCAGAG | Seq ID-60 |
| 31 | SNcOrf83-1F | TTCTTACTGGATGCCCGGTG | Seq ID-61 |
| | SNcOrf83-2R | TCTCACCAGCCCGTAGACT | Seq ID-62 |
| 32 | SNcOrf116-1F | GCTTCACAGCGTTGGGCTAT | Seq ID-63 |
| | SNcOrf116-2R | GAGATCCGCATAAGCAAGCC | Seq ID-64 |
| 33 | SNc342k-1F | TGAGGCAGAGTTTCGTCCTG | Seq ID-65 |
| | SNc342k-2R | AGCGCATAGCGGTTTTACGA | Seq ID-66 |
| 34 | SNc382k-1F | GCATTCCCATTGTAACGGCG | Seq ID-67 |
| | SNc383k-4R | TCTACTCGCCTGCCTACCTT | Seq ID-68 |
| 35 | SNcOrf89-1F | GTCGATCCAACGCCGTCTT | Seq ID-69 |
| | SNcOrf89-2R | TCCAATGCAGTAGCGGTACAC | Seq ID-70 |
| 36 | SNcOrf74c-1F | CTAAGGTGGAAGGGAATGGAGA | Seq ID-71 |
| | SNcOrf74c-2R | GAGCGAGGTTTGCCATTCTT | Seq ID-72 |
| 37 | SNcOrf91a-1F | GCTAGGGAGGAAGCCACTTT | Seq ID-73 |
| | SNcOrf91a-2R | CTCAGCTTCGCTCCTCAGTC | Seq ID-74 |
| 38 | SNcOrf72-1F | TCGCTTTCAGTTGAGGTGGA | Seq ID-75 |
| | SNcOrf72-2R | TAGATTGAGAGGCGGGAGGT | Seq ID-76 |
| 39 | SNcOrf77a-1F | GAAGCAGCGGAACATGAAGC | Seq ID-77 |
| | SNcOrf77a-2R | ATAAAGCTGCCCTCCCATCG | Seq ID-78 |
| 40 | SNcOrf77b-1F | TGCGGCGACAATACGTTCTT | Seq ID-79 |
| | SNcOrf77b-2R | ATCGACGGCATTCCAAAGGT | Seq ID-80 |
| 41 | SNc475k-1F | TCATAAGCAACCGAGCGGAA | Seq ID-81 |
| | SNc475k-2R | CCTTCACCATGCCTACCCTG | Seq ID-82 |

The results of analyzing the cytoplasm of each line of N. suaveolens, "Pt3", "P4", "Q15", and "pcf-CMS" using the primer sets designed for the regions presumed specific to N. suaveolens are shown in Table 6.

### [Table 6]

**Table 6: Results of analyzing cytoplasm using primers designed based on nucleotide sequence information of mitochondrial genome of "ms zhongyan100" (GenBank Accession No. KR071121)**

| Primer No. | Target region | D1 | D2 | D3 | D4 | D5 |
|---|---|---|---|---|---|---|
| 20 | 13144 - 13665 | N | 0 | 0 | 0 | 0 |
| 21 | 41300 - 41646 | N | 0 | N | 0 | 0 |
| 22 | 60180 - 60903 | N | 0 | 0 | 0 | 0 |
| 23 | 76232 - 76329 | N | 0 | 0 | 0 | 0 |
| 24 | 77082 - 77909 | N | P | P | P | P |
| 25 | 122416 - 122966 | N | 0 | 0 | 0 | 0 |
| 26 | 123155 - 123238 | N | 0 | 0 | 0 | 0 |
| 27 | 188636 - 189250 | N | 0 | N | 0 | 0 |
| 28 | 263700 - 263789 | N | 0 | 0 | 0 | 0 |
| 29 | 267332 - 267415 | N | 0 | N | 0 | 0 |
| 30 | 324267 - 325467 | N | 0 | N | N | 0 |
| 31 | 337452 - 337529 | N | 0 | 0 | 0 | 0 |
| 32 | 341103 - 341186 | N | 0 | 0 | 0 | 0 |
| 33 | 342384 - 342915 | N | 0 | 0 | 0 | 0 |
| 34 | 382068 - 383720 | N | 0 | N | N | 0 |
| 35 | 409611 - 409709 | N | P | P | P | P |
| 36 | 410354 - 410461 | N | 0 | 0 | 0 | 0 |
| 37 | 414449 - 414544 | N | 0 | 0 | 0 | 0 |
| 38 | 416107 - 416202 | N | 0 | 0 | 0 | 0 |
| 39 | 460790 - 460873 | N | 0 | 0 | 0 | 0 |
| 40 | 467403 - 467477 | N | 0 | 0 | 0 | 0 |
| 41 | 475128 - 475987 | N | 0 | 0 | 0 | 0 |

| | | | | | | |
|---|---|---|---|---|---|---|
| (Explanation of symbols in the table) D1: N. suaveolens D2: Pt3 (normal cytoplasm) D3: P4 D4: Q15 D5: pcf-CMS N: N. suaveolens type P: P. hybrida type 0: Not detected with the marker | | | | | | |

In the 22 primer sets designed this time, it was confirmed that specific DNA amplification occurred in N. suaveolens, specific amplification did not occur in Petunia having a normal cytoplasm except for a combination of primers Nos. 24 and 35, and petunia-specific amplification occurred in primers Nos. 24 and 35.

In "P4", amplification of DNA specific to N. suaveolens was observed in 5 primer sets (Primer Nos. 21, 27, 29, 30, and 34), and in "Q15", amplification of DNA specific to N. suaveolens was observed in 2 primer sets (Primer Nos. 30 and 34).
"pcf-CMS" is believed to have originated from a wild species of the genus Petunia and as with Table 4, no specific amplification of an N. suaveolens type was observed.

In "Q15", asymmetric back protoplast fusion was carried out using "P4" as a material, and it was concluded that elimination of the region of N. suaveolens occurred while leaving DNA regions involved in CMS, and that the mitochondrial genome was close to the mitochondrial genome of petunia.

"Q15", a novel Petunia cytoplasmic male sterile line, was further introduced into 51 diverse parent lines (BC1 to BC4), but fertility restoration was not observed.

Furthermore, no fertility restoration was observed in F1 test crosses of 91 combinations using these parent lines.

Therefore, the male sterility of "Q15" is considered extremely stable. On the other hand, as described in the "Background Art" section of the present description, in the known "pcf-CMS", restoration of fertility by a single dominant gene or a plurality of fertility restoration genes and restoration of fertility by environmental changes have been known (NPTL 3).

The reason why the cytoplasmic male sterility of the known "pcf-CMS" is unstable is thought to be because the known "pcf-CMS" originated from an interspecific hybrid in a plant of the genus Petunia, and a plurality of fertility restoration genes have appeared in a plant of the genus Petunia in the course of evolution. On the other hand, the reason why the cytoplasmic male sterility of the novel cytoplasmic male sterile Petunia "Q15" is stabilized was considered that since a cytoplasm from a tobacco plant is used, there is no fertility restoration gene in a distantly related plant of the genus Petunia.

### Example 3: Evaluation of growth ability at young seedling stage of novel petunia cytoplasmic male sterile line "Q15"

To confirm the usefulness of the novel Petunia cytoplasmic male sterile line "Q15" constructed by Example 2, a comparative test of the growth ability of young seedlings of "Pt3" having a normal cytoplasm and lines of "Q15" and "pcf-CMS" having a cytoplasm replaced by that of "Pt3" was carried out.

In the comparative test of the growth ability of young seedlings, "Pt3" having normal cells was used as a control, and a novel CMS line "Q15" produced by seven recurrent backcrossings (BC7) using "Pt3" as a recurrent parent and a known CMS line "pcf-CMS" were used. Since each CMS line is BC7, the nuclear genome was replaced by that of "Pt3" and each line has the same nuclear genome, and it is possible to compare differences in cytoplasm.

The seeds of each of the lines were seeded on a 128-hole cell tray and were cultivated in an artificial climate chamber set to a day temperature of 22°C, a night temperature of 15°C and a lighting time of 16 hours.

In order to evaluate the growth ability of young seedlings quantitatively, 30 days after the seeding the aboveground portion of young seedlings of each of the lines was cut at the ground surface and the weight per plant was measured.

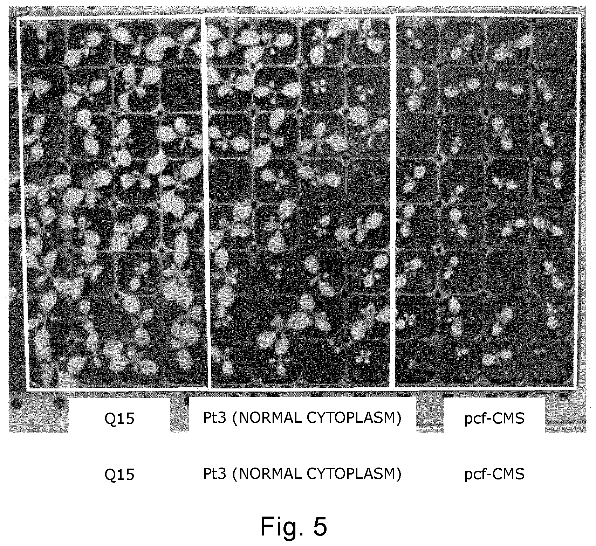

The results are shown in Table 7. In addition, in Fig. 5, a difference in the growth ability of each of the lines at that time is shown by photographs.

### [Table 7]

**Table 7: Results of comparing growth ability of young seedling between novel petunia cytoplasmic male sterile line and known pcf-CMS line**

| Line name | Generation | Cytoplasm | Test section | Frequency of seeding | Germination | Germination ratio | Average weight of aerial part (g) | Relative value of weight of aerial part1) |
|---|---|---|---|---|---|---|---|---|
| Pt3 | | N | A | 32 | 26 | 81% | 0.369 | 100 |
| Q15 | BC7 | 5 | A | 32 | 26 | 81% | 0.504 | 137 |
| pcf-CMS | BC7 | 5 | A | 32 | 28 | 88% | 0.143 | 39 |
| Pt3 | | N | B | 32 | 30 | 94% | 0.097 | 100 |
| Q15 | BC7 | 5 | B | 32 | 27 | 84% | 0.115 | 119 |
| pcf-CMS | BC7 | 5 | B | 32 | 28 | 88% | 0.046 | 47 |

| | | | | | | | | |
|---|---|---|---|---|---|---|---|---|
| (Explanation of symbols in the table) 1) A relative value of an average weight per plant of aerial parts of each CMS line when the average weight per head of aerial parts of petunia "Pt3" having a normal cytoplasm in the same test section is defined as 100 N: Normal cytoplasm S: Male sterile cytoplasm A: Artificial climate chamber (day temperature of 22°C, night temperature of 15°C, lighting time of 16 hours) B: Glass greenhouse (day temperature of 22°C, night temperature of 15°C, natural photoperiod) | | | | | | | | |

As shown in the column of test section A in Table 7, the "pcf-CMS" line had a relative value of the weight of the aboveground portion to that of "Pt3" of 39, and exhibited a low growth ability.

In contrast, the novel CMS line "Q15" had a relative value of the weight of aboveground portion to that of "Pt3" of "137", and exhibited an extremely high growth ability.

Next, a similar test was carried out in an environment of a glass greenhouse.

The glass greenhouse (located in Kakegawa-shi, Shizuoka-ken, Japan) was set to a day temperature of 22°C and a night temperature of 15°C, and the seeds of each of the lines were seeded on a 128-hole cell tray on April 1, 2020, and were cultivated.

In order to evaluate the growth ability of a young seedling quantitatively, 30 days after seeding, the aboveground portion of young seedlings of each of the lines was cut at the ground surface and the weight per plant was measured.

The results are also shown in Table 7.

As shown in the column of test section B in Table 7, the "pcf-CMS" line had a relative value of the weight of aboveground portion to that of "Pt3" of "47", and exhibited a low growth ability.

In contrast, the novel CMS line "Q15" had a relative value of the weight of aboveground portion to that of "Pt3" of "119", and exhibited a high growth ability.

From the above results, while the known "pcf-CMS" line exhibited deterioration in the growth ability at a young seedling stage in both environments, the novel petunia cytoplasmic male sterile line "Q15" exhibited a growth ability exceeding that of "Pt3" having a normal cytoplasm in both environments, and the usefulness was confirmed.

The seeds of the novel Petunia cytoplasmic male sterile line "Q15" have been internationally deposited (originally deposited) on August 28, 2020 at International Patent Organism Depositary of the National Institute of Technology and Evaluation located at Room 120, 2-5-8 Kazusa Kamatari, Kisarazu-shi, Chiba-ken, Japan (Indications for identification given by depositor: SSC-PET-20-001, Accession No.: FERM BP-22398).

### Example 4: Introduction of novel petunia cytoplasmic male sterility into intergeneric hybrid plant

To introduce the novel Petunia cytoplasmic male sterility using N. suaveolens constructed in the present invention into a fertile intergeneric hybrid plant of Petunia (Petunia × Calibrachoa), recurrent backcrossing was carried out using a novel Petunia cytoplasmic male sterile line as a seed parent (nonrecurrent parent) and seven fertile intergeneric hybrid plants of Petunia as pollen parents (recurrent parents) to carry out cytoplasmic replacement.

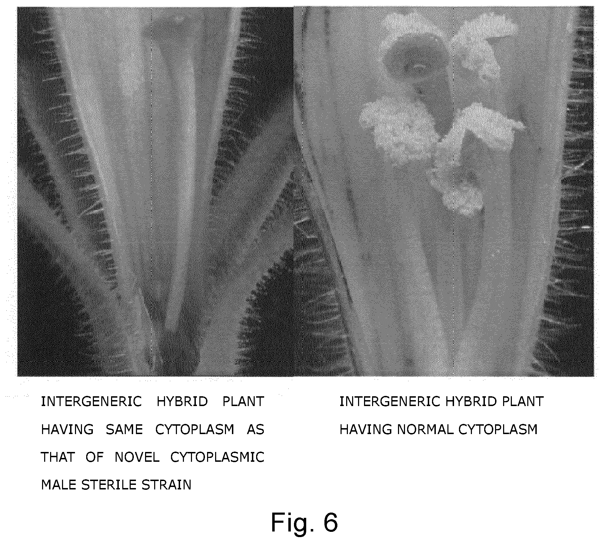

In the intergeneric hybrid plants whose cytoplasm was replaced by the cytoplasm of the novel cytoplasmic male sterile line by recurrent backcrossing, anthers were degenerated and no pollen grains were formed, as shown in Fig. 6.

It was confirmed that all 112 individuals of 7 lines of the intergeneric hybrid plants tested were male sterile, and the cytoplasmic male sterility of the novel cytoplasmic male sterile line stably exhibited cytoplasmic male sterility also in the intergeneric hybrid plants.

Therefore, it was confirmed that the cytoplasm of the novel CMS line from N. suaveolens, according to the present invention, can be introduced into a closely related intergeneric hybrid plant and exhibit cytoplasmic male sterility.

[Sequence Listing]

## Claims

1. A cytoplasmic male sterile plant of the genus Petunia comprising, in a mitochondrial genome thereof, a DNA molecule originated from a mitochondrial genome of a tobacco plant, or a hybrid plant with the cytoplasmic male sterile plant of the genus Petunia, or a progeny thereof.

2. The cytoplasmic male sterile plant of the genus Petunia or the hybrid plant with the cytoplasmic male sterile plant of the genus Petunia, or the progeny thereof according to claim 1, wherein the tobacco plant is Nicotiana suaveolens.

3. The cytoplasmic male sterile plant of the genus Petunia or the hybrid plant with the cytoplasmic male sterile plant of the genus Petunia, or the progeny thereof according to claim 1 or 2, wherein the cytoplasmic male sterile plant of the genus Petunia is originated from Petunia hybrida or an interspecific hybrid plant thereof.

4. The cytoplasmic male sterile plant of the genus Petunia or the hybrid plant with the cytoplasmic male sterile plant of the genus Petunia, or the progeny thereof according to any one of claims 1 to 3, wherein the cytoplasmic male sterile plant of the genus Petunia is originated from one produced by asymmetric protoplast fusion using a tobacco plant as a cytoplasm donor parent.

5. The cytoplasmic male sterile plant of the genus Petunia or the hybrid plant with the cytoplasmic male sterile plant of the genus Petunia, or the progeny thereof according to any one of claims 1 or 4, wherein the hybrid plant with the cytoplasmic male sterile plant of the genus Petunia is originated from an intergeneric hybrid plant between a plant of the genus Petunia and a plant of the genus Calibrachoa.

6. The cytoplasmic male sterile plant of the genus Petunia or the hybrid plant with the cytoplasmic male sterile plant of the genus Petunia, or the progeny thereof according to any one of claims 1 to 5, comprising a mitochondrial genome originated from a plant identified by Accession No. FERM BP-22398.

7. The cytoplasmic male sterile plant of the genus Petunia or the hybrid plant with the cytoplasmic male sterile plant of the genus Petunia, or the progeny thereof according to any one of claims 1 to 6, wherein at least one of mitochondrial DNA regions is a Nicotiana suaveolens type where said regions are identified by a mitochondrial genome marker using one or more primers selected from the group consisting of primers contained in a primer set having nucleotide sequences set forth in SEQ ID NOs: 59 and 60 and a primer set having nucleotide sequences set forth in SEQ ID NOs: 67 and 68.

8. The cytoplasmic male sterile plant of the genus Petunia or the hybrid plant with the cytoplasmic male sterile plant of the genus Petunia, or the progeny thereof according to any one of claims 1 to 7, comprising a mitochondrial genome of a plant identified by Accession No. FERM BP-22398.

9. The cytoplasmic male sterile plant of the genus Petunia or the hybrid plant with the cytoplasmic male sterile plant of the genus Petunia, or the progeny thereof according to any one of claims 1 to 7, which is identified by Accession No. FERM BP-22398.

10. The cytoplasmic male sterile plant of the genus Petunia or the hybrid plant with the cytoplasmic male sterile plant of the genus Petunia, or the progeny thereof according to any one of claims 1 to 9, which is produced by asymmetric protoplast fusion using, as a cytoplasm donor parent, a cytoplasmic male sterile plant of the genus Petunia or a hybrid plant with a cytoplasmic male sterile plant of the genus Petunia, having a mitochondrial genome of a plant identified by Accession No. FERM BP-22398, and using, as a cytoplasm acceptor parent, a plant of the genus Petunia or a hybrid plant with a plant of the genus Petunia, having a normal cytoplasm.

11. A part of a plant body of the cytoplasmic male sterile plant of the genus Petunia or the hybrid plant with the cytoplasmic male sterile plant of the genus Petunia, or the progeny thereof as defined in any one of claims 1 to 10.

12. A seed of the cytoplasmic male sterile plant of the genus Petunia or the hybrid plant with the cytoplasmic male sterile plant of the genus Petunia, or the progeny thereof as defined in any one of claims 1 to 10.

13. A mitochondrial genome contained in the cytoplasmic male sterile plant of the genus Petunia or the hybrid plant with the cytoplasmic male sterile plant of the genus Petunia, or the progeny thereof as defined in any one of claims 1 to 10, a part of the plant body as defined in claim 11, or the seed as defined in claim 12.

14. A method for producing a cytoplasmic male sterile plant of the genus Petunia or a hybrid plant with the cytoplasmic male sterile plant of the genus Petunia, or a progeny thereof, the method comprising a step of carrying out asymmetric protoplast fusion using a tobacco plant as a cytoplasm donor parent and using a plant of the genus Petunia or a hybrid plant with a plant of the genus Petunia, having a normal cytoplasm, as a cytoplasm acceptor parent.

15. The production method according to claim 14, wherein said tobacco plant is Nicotiana suaveolens.

16. A method for producing a cytoplasmic male sterile plant of the genus Petunia having an improved mitochondrial genome or a hybrid plant with the cytoplasmic male sterile plant of the genus Petunia, or a progeny thereof, the method comprising a step of carrying out asymmetric protoplast fusion using a cytoplasmic male sterile plant of the genus Petunia having, in a mitochondrial genome thereof, a DNA molecule originated from a mitochondrial genome of a tobacco plant, or a hybrid plant with the cytoplasmic male sterile plant of the genus Petunia, or a progeny thereof as a cytoplasm donor parent and using a plant of the genus Petunia or a hybrid plant with a plant of the genus Petunia, having a normal cytoplasm, as a cytoplasm acceptor parent.

17. The production method according to any one of claims 14 to 16, wherein the cytoplasmic male sterile plant of the genus Petunia is originated from Petunia hybrida or an interspecific hybrid plant thereof.

18. The production method according to any one of claims 14 to 17, wherein the hybrid plant with the cytoplasmic male sterile plant of the genus Petunia is originated from an intergeneric hybrid plant between a plant of the genus Petunia and a Calibrachoa plant.

19. A method for producing a first filial generation seed, comprising the steps of: carrying out crossbreeding using, as a seed parent, the cytoplasmic male sterile plant of the genus Petunia or the hybrid plant with the cytoplasmic male sterile plant of the genus Petunia, or the progeny thereof as defined in any one of claims 1 to 10 and using, as a pollen parent, a plant of the genus Petunia capable of being crossbred with the plant and an intergeneric hybrid originated from the plant of the genus Petunia and then producing a first filial generation seed from said seed parent after the crossbreeding.

20. A first filial generation seed produced by a method as defined in claim 19, a first filial generation plant grown from the seed or a progeny thereof, or a part of a plant body of the first filial generation plant or the progeny thereof.

21. A method for producing a plant of the genus Petunia exhibiting cytoplasmic male sterility and an intergeneric hybrid plant originated therefrom, the method comprising the steps of carrying out recurrent backcrossing of an arbitrary plant of the genus Petunia and an intergeneric hybrid plant originated therefrom to a cytoplasmic male sterile plant of the genus Petunia or a hybrid plant with the cytoplasmic male sterile plant of the genus Petunia, or a progeny thereof as defined in any one of claims 1 to 10 to achieve cytoplasmic replacement.

22. A primer set comprising a primer having a nucleotide sequence set forth in SEQ ID NO: 59 and a primer having a nucleotide sequence set forth in SEQ ID NO: 60.

23. A primer set comprising a primer having a nucleotide sequence set forth in SEQ ID NO: 67 and a primer having a nucleotide sequence set forth in SEQ ID NO: 68.
